# EUROPÄISCHE PATENTANMELDUNG

(11) **EP 3 929 352 A1**
(43) Veröffentlichungstag der Anmeldung: **29.12.2021**
(21) Anmeldenummer: 21178705.6
(22) Anmeldetag: 10.06.2021
(51) Int. Cl.: D06N 3/00, D06N 3/14

(54) **VERWENDUNG VON ZWEISCHWÄNZIGEN, LANGKETTIGEN ANIONTENSIDEN IN WÄSSRIGEN POLYURETHANDISPERSIONEN**

(30) Priorität: 24.06.2020 EP 20181876
(71) Anmelder: Evonik Operations GmbH, 45128 Essen (DE)
(72) Erfinder: Klostermann, Michael, 45141 Essen (DE); Feldmann, Kai-Oliver, 45133 Essen (DE); von Hof, Jan Marian, 44789 Bochum (DE); Arnold, Sina, 46244 Bottrop (DE); Jansen, Marvin, 45131 Essen (DE)
(74) Vertreter: Evonik Patent Association

(57) **Zusammenfassung**

Die Verwendung von zweischwänzigen, langkettigen Aniontensiden als Additive in wässrigen PolymerDispersionen zur Herstellung poröser Polymerbeschichtungen, bevorzugt zur Herstellung von porösen Polyurethanbeschichtungen wird beschrieben.

## Beschreibung

Die vorliegende Erfindung liegt auf dem Gebiet der Kunststoffbeschichtungen und Kunstleder.

Sie betrifft insbesondere die Herstellung poröser Polymerbeschichtungen, bevorzugt poröser Polyurethanbeschichtungen unter Verwendung von zweischwänzigen, langkettigen Aniontensiden als Additive.

Mit Kunststoffen beschichtete Textilien, wie z.B. Kunstleder, bestehen in der Regel aus einem textilen Träger, aufweichen eine poröse Polymerschicht laminiert wird, welche wiederum mit einer Deckschicht bzw. einem Decklack überzogen ist.

Die poröse Polymerschicht weist hierbei vorzugsweise Poren im Mikrometer-Bereich auf, ist luftdurchlässig und damit atmungsaktiv, d.h. durchlässig für Wasserdampf, jedoch wasserbeständig. Bei der porösen Polymerschicht handelt es sich oftmals um poröses Polyurethan. Zur umweltfreundlichen Herstellung von PU-basiertem Kunstleder wurde vor geraumer Zeit ein Verfahren entwickelt, welches auf wässrigen Polyurethan-Dispersionen, sogenannten PUDs, basiert. Diese bestehen in der Regel aus in Wasser dispergierten Polyurethan-Mikropartikeln, der Festkörpergehalt liegt meist im Bereich von 30 - 60 Gew.-%. Zur Herstellung einer porösen Polyurethan-Schicht werden diese PUDs mechanisch aufgeschäumt, auf einen Träger beschichtet (Schichtdicken typischer Weise zwischen 300 - 2000 µm) und anschließend bei erhöhter Temperatur getrocknet. Während dieses Trocknungsschritts verdampft das im PUD-System enthaltene Wasser, wodurch es zu einer Verfilmung der Polyurethan-Partikel kommt. Um die mechanische Festigkeit des Films weiter zu erhöhen können dem PUD-System während des Herstellungsprozesses zusätzlich hydrophile (Poly)lsocyanate oder Carbodiimide zugesetzt werden, welche während des Trocknungsschritts mit freien, an der Oberfläche der Polyurethan-Partikel vorhandenen OH-Resten reagieren können und so zu einer zusätzlichen Vernetzung des Polyurethan-Films führen.

Sowohl die mechanischen als auch die haptischen Eigenschaften so hergestellter PUD-Beschichtungen werden maßgeblich von der Zellstruktur des porösen Polyurethanfilms bestimmt. Weiterhin hat die Zellstruktur des porösen Polyurethanfilms Einfluss auf die Luftdurchlässigkeit bzw. Atmungsaktivität des Materials. Besonders gute Eigenschaften lassen sich hierbei mit möglichst feinen, homogen verteilten Zellen erzielen. Zur Beeinflussung der Zellstruktur während des oben beschrieben Herstellverfahrens ist es üblich dem PUD-System vor bzw. während des mechanischen Aufschäumens Schaumstabilisatoren zuzusetzen. Entsprechende Stabilisatoren führen zum einen dazu, dass sich während des Aufschäumvorgangs ausreichende Mengen Luft ins PUD-System einschlagen lassen. Zum andern haben die Schaumstabilisatoren einen direkten Einfluss auf die Morphologie der erzeugten Luftblasen. Auch die Stabilität der Luftblasen wird maßgeblich durch die Art des Stabilisators beeinflusst. Dies ist insbesondere während derTrocknung geschäumter PUD-Beschichtungen wichtig, da sich hierdurch Trocknungsdefekte wie Zellvergröberung oder Trocknungsrisse unterbinden lassen.

In der Vergangenheit kamen bereits verschiedene Schaumstabilisatoren im oben beschrieben PUD-Verfahren zum Einsatz. In den Schriften US 2015/0284902 A1 oder US 2006 0079635 A1 wird beispielsweise die Verwendung von Ammonium-Stearat-basierten Schaumstabilisatoren beschrieben. Der Einsatz entsprechender Ammonium-Stearat-basierter Stabilisatoren geht jedoch mit einer Reihe an Nachteilen einher. Ein wesentlicher Nachteil ist hierbei, dass Ammonium-Stearat im fertigen Kunstleder eine sehr hohe Migrierfähigkeit aufweist. Dies führt dazu, dass sich mit der Zeit Tensidmoleküle an der Oberfläche des Kunstleders anreichern, wodurch es zu weißen Verfärbungen an der Oberfläche des Leders kommen kann. Des Weiteren kann durch diese Tensidmigration ein als unangenehm empfundener Schmierfilm an der Oberfläche des Kunstleders entstehen, insbesondere dann, wenn entsprechende Materialien in Kontakt mit Wasser kommen.

Ein weiterer Nachteil von Ammonium-Stearat ist, dass es in Kontakt mit kalkhaltigem Wasser unlösliche Kalkseifen bildet. Bei Kontakt von auf Basis von Ammonium-Stearat hergestelltem Kunstleder mit kalkhaltigem Wasser können so weiße Aufblühungen an der Kunstleder-Oberfläche entstehen, was insbesondere bei dunkel eingefärbtem Leder unerwünscht ist.

Noch ein Nachteil von Ammonium-Stearat-basierten Schaumstabilisatoren ist, dass sie zwar ein effizientes Aufschäumen von wässrigen Polyurethan-Dispersionen erlauben, hierbei jedoch oftmals zu einer recht groben und irregulären Schaumstruktur führen. Dies kann sich negativ auf die optischen und haptischen Eigenschaften des fertigen Kunstleders auswirken.

Noch ein Nachteil von Ammonium-Stearat ist, dass die erzeugten PUD-Schäume oftmals eine unzureichende Stabilität aufweisen, was zu Nachteilen bei deren Verarbeitung, insbesondere bei der Trocknung der PUD-Schäume bei erhöhten Temperaturen, führen kann. Eine Folge hiervon wäre beispielsweise, dass entsprechende Schäume relativ schonend und langsam getrocknet werden müssen, was wiederum zu längeren Prozesszeiten bei der Kunstlederherstellung führt.

Als Alternative zu Ammonium-Stearat-basierten Schaumstabilisatoren konnten in der Vergangenheit Polyolester sowie Polyolether als wirkungsvolle Schaumadditive für wässrige Polyurethan-Dispersionen identifiziert werden. Diese Strukturen sind beispielsweise in den Schriften EP 3487945 A1 bzw. WO2019042696A1 beschrieben. Gegenüber Ammonium-Stearat weisen Polyolester bzw. Polyolether den wesentlichen Vorteil auf, dass sie nicht bzw. nur schwach im fertigen Kunstleder migrieren und somit nicht zu unerwünschten Oberflächenverfärbungen führen. Außerdem sind Polyolester bzw. Polyolether nicht anfällig gegenüber kalkhaltigem Wasser.

Ein weiterer Vorteil von Polyolester und Polyolethern gegenüber Ammonium-Stearat-basierten Schaumstabilisatoren ist zudem, dass sie oftmals zu einer deutlich feineren und homogeneren Schaumstruktur führen, was sich vorteilhaft auf die Eigenschaften von mit diesen Substanzen hergestellten Kunstledermaterialien auswirkt. Auch führen Polyolester und Polyolether oftmals zu deutlich stabileren PUD-Schäumen, was wiederum prozesstechnische Vorteile bei der Kunstlederherstellung mit sich bringt.

Trotz dieser Vorteile sind Polyolester und Polyolether auch nicht völlig frei von potentiellen Nachteilen. Ein potentieller Nachteil hierbei ist, dass die schaumstabilisierende Wirkung dieser Verbindungsklassen durch die Anwesenheit weiterer, im PUD-System enthaltener Co-Tenside unter Umständen beeinträchtigt werden kann. Insbesondere bei der Herstellung von wässrigen Polyurethan-Dispersionen ist die Verwendung von Co-Tensiden jedoch nicht unüblich. Co-Tenside werden in diesem Zusammenhang zur verbesserten Dispergierung von Polyurethan-Prepolymeren in Wasser eingesetzt und verbleiben in der Regel im finalen Produkt. Während des mechanischen Aufschäumens wässriger Polyurethandispersion, welche Polyolester bzw. Polyolether als Schaumadditive enthalten, können sich entsprechende Co-Tenside unter Umständen negativ auf das Aufschäumverhalten des Systems auswirken. Hierdurch kann unter Umständen nur wenig bis gar keine Luft ins System eingeschlagen werden kann; die erhaltene Schaumstruktur könnte dann leiden. Co-Tenside können sich außerdem negativ auf die Stabilität der erzeugten Schäume auswirken, wodurch es während der Verarbeitung des geschäumten PUD-Systems zu Schaumalterung kommen kann, was wiederum zu Fehlstellen und Defekten in den hergestellten Schaumbeschichtungen führt.

Ein weiterer potentieller Nachteil ist, dass PUD-Systeme, welche Polyolester bzw. Polyolether als Schaumadditive enthalten, oft sehr hohe Scherenergien für ein effizientes Aufschäumen benötigen. Dies wiederum kann unter Umständen Limitierungen und prozesstechnische Nachteile mit sich bringen.

Aufgabe der vorliegenden Erfindung war daher die Bereitstellung von Additiven zur Herstellung von PUD-basierten Schaumsystemen und Schaumbeschichtungen, welche ein effizientes Aufschäumen von PUD-Systemen ermöglichen und nicht die im Stand der Technik aufgeführten Nachteile aufweisen. Überraschender Weise wurde gefunden, dass zweischwänzige, langkettige Aniontenside die Lösung der genannten Aufgabe ermöglichen.

Gegenstand der vorliegenden Erfindung ist daher die Verwendung von zweischwänzigen, langkettigen Aniontensiden als Additive, vorzugsweise als Schaumadditive, in wässrigen Polymer-Dispersionen, vorzugsweise wässrigen Polyurethan-Dispersionen, zur Herstellung poröser Polymerbeschichtungen, bevorzugt zur Herstellung von porösen Polyurethanbeschichtungen.

Die erfindungsgemäße Verwendung zweischwänziger, langkettiger Aniontensiden hat hierbei überraschender Weise mannigfaltige Vorteile.

Ein Vorteil ist, dass zweischwänzige, langkettige Aniontenside ein besonders effizientes Aufschäumen von wässrigen PUD-Systemen ermöglichen. Die so hergestellten Schäume zeichnen sich hierbei durch eine außergewöhnlich feine Porenstruktur mit besonders homogener Zellverteilung aus, was sich wiederum sehr vorteilhaft auf die mechanischen und haptischen Eigenschaften der porösen Polymerbeschichtung auswirkt, welche auf Basis dieser Schäume hergestellt werden. Des Weiteren kann hierdurch die Luftdurchlässigkeit bzw. Atmungsaktivität der Beschichtung verbessert werden.

Ein weiterer Vorteil ist, dass zweischwänzige, langkettige Aniontenside auch bei relativ niedrigen Schergeschwindigkeiten ein effizientes Aufschäumen von PUD-Systemen ermöglichen, was zu weniger Limitierungen und einer breiteren Verarbeitbarkeit während der Kunstlederherstellung führt.

Noch ein Vorteil ist, dass zweischwänzige, langkettige Aniontenside die Herstellung besonders stabiler Schäume ermöglichen. Dies wirkt sich zum einen vorteilhaft auf deren Verarbeitbarkeit aus. Zum andern hat die erhöhte Schaumstabilität den Vorteil, dass sich während der Trocknung entsprechender Schäume Trocknungsdefekte wie Zellvergröberung oder Trocknungsrisse vermeiden lassen. Darüber hinaus erlaubt die verbesserte Schaumstabilität ein schnelleres Trocknen der Schäume, was prozesstechnische Vorteile sowohl aus ökologischer als auch aus ökonomischer Sicht bietet.

Noch ein Vorteil ist, dass die Wirksamkeit von zweischwänzigen, langkettigen Aniontensiden nicht bzw. kaum von im PUD-System enthaltenen Co-Tensiden beeinträchtigt wird. So ermöglichen die erfindungsgemäßen Tensidformulierungen auch im Falle von Co-Tensid-haltigen PUD-Systemen ein effizientes Aufschäumen des Systems, sowie die Bildung von feinen und homogenen, sowie gleichzeitig äußerst stabilen Schäumen.

Noch ein Vorteil ist, dass die erfindungsgemäßen zweischwänzigen, langkettigen Aniontenside im fertigen Kunstleder nicht bzw. kaum migrierfähig sind und so nicht zu unerwünschten Oberflächenverfärbungen oder Aufblühungen führen. Des Weiteren sind die erfindungsgemäßen Tenside nicht bzw. kaum anfällig gegenüber kalkhaltigem Wasser.

Der Begriff zweischwänziges, langkettiges Aniontensid umfasst im Sinne der gesamten vorliegenden Erfindungen Tenside, die eine anionische hydrophile Kopfgruppe sowie zwei gleiche oder verschiedene langkettige hydrophobe Kohlenwasserstoff-Reste aufweisen. Langkettig bedeutet in diesem Zusammenhang, dass die hydrophoben Kohlenwasserstoffreste aus mindestens 12, bevorzugt mindestens 14 Kohlenstoffatomen noch mehr bevorzugt aus mindestens 16 Kohlenstoffatomen bestehen.

Der Begriff Co-Tensid umfasst im Sinne der gesamten vorliegenden Erfindung zusätzliche Tenside, welche neben den erfindungsgemäßen zweischwänzigen, langkettigen Aniontensiden in der Polymerdisperion enthalten sein können. Insbesondere fallen hierunter Tenside, die während der Herstellung der Polymerdispersion verwendet werden. Beispielsweise erfolgt die Herstellung von Polyurethan-Dispersionen oftmals durch Synthese eines PU-Prepolymers, welches in einem zweiten Schritt in Wasser dispergiert und anschließend mit einem Kettenverlängerer abreagiert wird. Zur verbesserten Dispergierung des Prepolymers in Wasser können hierbei Co-Tenside verwendet werden. Im Rahmen der vorliegenden Erfindung handelt es sich bei Co-Tensiden vorzugsweise um anionische Co-Tenside.

Die Erfindung wird nachfolgend weiter und beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere in Bezug auf den Sachverhalt, in dessen Zusammenhang das Dokument zitiert wurde, vollständig zum Offenbarungsgehalt der vorliegenden Erfindung gehören. Bei Prozentangaben handelt es sich, wenn nicht anders angegeben, um Angaben in Gewichtsprozent. Werden nachfolgend Parameter angegeben, die durch Messung bestimmt wurden, so wurden die Messungen, wenn nicht anders angegeben, bei einer Temperatur von 25 °C und einem Druck von 101325 Pa durchgeführt. Werden in der vorliegenden Erfindung chemische (Summen-)Formeln verwendet, so können die angegebenen Indizes sowohl absolute Zahlen als auch Mittelwerte darstellen. Bei polymeren Verbindungen stellen die Indizes vorzugsweise Mittelwerte dar. In der vorliegenden Erfindung dargestellte Struktur und Summenformeln stehen stellvertretend für alle durch unterschiedliche Anordnung der sich wiederholenden Einheiten denkbaren Isomere.

Im Rahmen der vorliegenden Erfindung sind solche zweischwänzigen, langkettigen Aniontenside bevorzugt, welche aus einer anionischen Kopfgruppe sowie langkettigen hydrophoben Resten bestehen. Die hydrophoben Reste können hierbei unabhängig voneinander gleiche oder verschiedene einwertige aliphatische oder aromatische, gesättigte oder ungesättigte Kohlenwasserstoffrest mit 12 bis 40 C-Atomen, bevorzugt 14 bis 30, besonders bevorzugt mit 16 bis 24 Kohlenstoffatomen sein. Bevorzugt basiert die anionische Kopfgruppe hierbei entweder auf organischen Carboxylaten oder anionischen Schwefel- oder Phosphorverbindungen. Besonders bevorzugt weist die anionische Kopfgruppe Struktureinheiten ausgewählt aus der Gruppe der Carboxylate, der Phosphate, der Phosphonate, der Phosiphinate, der Sulfate sowie der Sulfonate auf.

Im Rahmen der vorliegenden Erfindung sind insbesondere zweischwänzige, langkettige Aniontenside bevorzugt, welche der allgemeinen Formel (I) entsprechen, 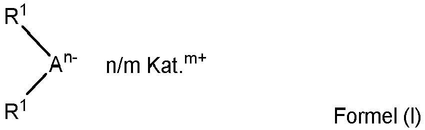 wobei die Reste R¹ unabhängig voneinander gleiche oder verschiedene einwertige gesättigte oder ungesättigte aliphatische oder aromatische Kohlenwasserstoffreste mit 12 bis 40 C-Atomen, bevorzugt 14 bis 30, besonders bevorzugt mit 16 bis 24 Kohlenstoffatomen sind, und wobei Aⁿ⁻ die anionische Kopfgruppe des Tensids ist, welche n negative Ladungen trägt, wobei n = 1 - 3, mehr bevorzugt 1 - 2, noch mehr bevorzugt gleich 1, und wobei Kat.ⁿ⁺ das kationische Gegenion ist, welches n positive Ladungen trägt, wobei n = 1 - 10, bevorzugt 1 - 5, mehr bevorzugt 1 - 2, noch mehr bevorzugt gleich 1.

Die Anbindung der Reste R¹ an die anionische Kopfgruppe kann hierbei über vielerlei Verknüpfungsmotive erfolgen, beispielsweise über, aber nicht beschränkt auf, Ester-, Amide-, Ether, Carbonat- oder Silikonbindungen. Weiterhin kann die Anbindung der Reste R¹ an die anionische Kopfgruppe über Polyoxyalkylen-, bevorzugt über Polyoxyethylen- und Polyoxypropylen-Brücken erfolgen. Besonders bevorzugt sind in diesem Zusammenhang Polyoxyalkylen-, bevorzugt über Polyoxyethylen- und Polyoxypropylen-Brücken, mit nicht mehr als 10, bevorzugt nicht mehr als 7, mehr bevorzugt nicht mehr bevorzugt als 5, noch mehr bevorzugt nicht mehr als 3 Alkoxyeinheiten. In diesem Fall sind entsprechende Polyoxyalkylen-, bevorzugt über Polyoxyethylen- und Polyoxypropylen-Brücken als Teil der anionischen Kopfgruppe zu betrachten. Kombinationen der genannten Verknüpfungs- und Verbrückungsmotive sind im Rahmen der vorliegen Erfindung ebenfalls bevorzugt.

Im Rahmen der vorliegenden Erfindung ist bevorzugt, wenn die anionische Kopfgruppe Aⁿ⁻ Strukturmerkale organischer Carboxylate oder anionischen Schwefel- oder Phosphorverbindungen aufweist. Besonders bevorzugt weist die anionische Kopfgruppe herbei Struktureinheiten ausgewählt aus der Gruppe der Carboxylate, der Phosphate, der Phosphonate, der Phosphinate, der Sulfate sowie der Sulfonate auf.

Bevorzugte Reste R¹ leiten sich hierbei von langkettigen Alkoholen, Carbonsäuren oder Alkylaminen mit mindestens 12 bis 40 C-Atomen, bevorzugt 14 bis 30, besonders bevorzugt mit 16 bis 24 Kohlenstoffatomen, sowie Mischungen dieser Substanzen ab.

Leitet sich der Rest R¹ von langkettigen Alkoholen ab, so sind dies bevorzugt Laurylalkohol (1-Dodecanol), Myristylalkohol (1-Tetradecanol), Cetylalkohol (1-Hexadecanol), Margarylalkohol (1-Heptadecanol), Stearylalkohol (1-Octadecanol), Arachidylalkohol (1-Eicosanol), Behenylalkohol (1-Docosanol), Lignocerylalkohol (1-Tetracosanol), Cerylalkohol (1-Hexacosanol), Montanylalkohol (1-Octacosanol), Melissylalkohol (1-Triacontanol), Palmitoleylalkohol (cis-9-Hexadecen-1-ol), Oleylalkohol (cis-9-Octadecen-1-ol) und/oder Elaidylalkohol (trans-9-Octadecen-1-ol) sowie Mischungen dieser Substanzen, wobei Palmitylalkohol und Stearylalkohol sowie Mischungen dieser beiden Substanzen besonders bevorzugt sind. Erfindungsgemäße bevorzugt sind hierbei sowohl die reinen Alkohole, sowie deren technische Qualitäten, welche Kettenlängenverteilungen aufweisen bzw. Gemische aus Fettsäuren unterschiedlicher Kettenlänge enthalten.

Leitet sich der Rest R¹ von langkettigen Carbonsäuren ab, so sind diese bevorzugt ausgewählt aus Laurinsäure (Dodecansäure), Myristinsäure (Tetradecansäure), Palmitinsäure (Hexadecansäure), Stearinsäure (Octadecansäure), Arachinsäure (Eicosansäure), Behensäure (Docosansäure), Lignocerinsäure (Tetracosansäure), Palmitölsäure ((Z)-9-Hexadecensäure), Ölsäure ((Z)-9-Hexadecensäure), Elaidinsäure ((E)-9-Octadecensäure), cis-Vaccensäure ((Z)-11-Octadecensäure), Linolsäure ((9Z,12Z)-9,12-Octadecadiensäure), alpha-Linolensäure ((9Z,12Z,15Z)-9,12,15-Octadecatriensäure), gamma-Linolensäure ((6Z,9Z,12Z)-6,9,12-Octadecatriensäure), Di-homo-gamma-Linolensäure ((8Z,11Z,14Z)-8,11,14-Eicosatriensäure), Arachidonsäure ((5Z,8Z,11Z,14Z)-5,8,11,14- Eicosatetraensäure), Erucasäure ((Z)-13-Docosensäure), Nervonsäure ((Z)-15-Tetracosensäure), Rizinolsäure, Hydroxystearinsäure und/oder Undecenylsäure, sowie deren Mischungen, wie z.B. Rapsölsäure, Soyafettsäure, Sonnenblumenfettsäure, Erdnussfettsäure und Tallölfettsäure. Ganz besonders bevorzugt sind Palmitinsäure und Stearinsäure sowie insbesondere die Gemische dieser Substanzen. Erfindungsgemäße bevorzugt sind hierbei sowohl die reinen Carbonsäuren, sowie deren technische Qualitäten, welche Kettenlängenverteilungen aufweisen bzw. Gemische aus Fettsäuren unterschiedlicher Kettenlänge enthalten.

Leitet sich der Rest R¹ von langkettigen Aminen ab, so sind insbesondere Laurylamin (1-Dodecylamin), Myristylamin (1-Tetradecylamin), Cetylamin (1-Hexadecylamin), Margarylamin (1-Heptadecylamin), Stearylamin (1-Octadecylamin), Arachidylamin (1-Eicosylamin), Behenylamin (1-Docosylamin), Lignocerylamin (1-Tetracosylamin), Cerylamin (1-Hexacosylamin), Montanylamin (1-Octacosylamin), Melissylamin (1-Triacontylamin), Palmitoleylamin (cis-9-Hexadecenylamin), Oleylamin (cis-9-Octadecenylamin) und/oder Elaidylamin (trans-9-Octadecenylamin) sowie Mischungen, wobei Palmitylamin und Stearylamin sowie Mischungen dieser beiden Substanzen besonders bevorzugt sind. Erfindungsgemäße bevorzugt sind hierbei sowohl die reinen Amine, sowie deren technische Qualitäten, welche Kettenlängenverteilungen aufweisen bzw. Gemische aus Fettsäuren unterschiedlicher Kettenlänge enthalten.

Quellen für die oben beschriebenen langkettigen Alkohole, Amine und Carbonsäuren können pflanzliche oder tierische Fett, Öle oder Wachse sein. Beispielsweise können verwendet werden: Schweineschmalz, Rindertalg, Gänsefett, Entenfett, Hühnerfett, Pferdefett, Walöl, Fischöl, Palmöl, Olivenöl, Avokadoöl, Samenkernöle, Kokosöl, Palmkernöl, Kakaobutter, Baumwollsamenöl, Kürbiskernöl, Maiskeimöl, Sonnenblumenöl, Weizenkeimöl, Traubenkernöl, Sesamöl, Leinsamenöl, Sojabohnenöl, Erdnussöl, Lupinöl, Rapsöl, Senföl, Rizinusöl, Jatropaöl, Walnussöl, Jojobaöl, Lecithin z.B. auf Basis von Soja, Raps, oder Sonnenblumen, Knochenöl, Klauenöl, Borretschöl, Lanolin, Emuöl, Hirschtalg, Murmeltieröl, Nerzöl, Distelöl, Hanföl, Kürbisöl, Nachtkerzenöl, Tallöl, sowie Carnaubawachs, Bienenwachs, Candelillawachs, Ouricuriwachs, Zuckerrohrwachs, Retamowachs, Carandaywachs, Raffiawachs, Espartowachs, Alfalfawachs, Bambuswachs, Hempwachs, Douglastannenwachs, Korkwachs, Sisalwachs, Flachswachs, Baumwollwachs, Dammarwachs, Teewachs, Kaffeewachs, Reiswachs, Oleanderwachs, Bienenwachs und/oder Wollwachs.

Weiterhin sind im Rahmen der vorliegenden Erfindung solche zweischwänzige, langkettige Aniontenside bevorzugt, welche der allgemeinen Formel (II) entsprechen,

R²-Aⁿ- n/m Kat.^{m+} Formel (II)

wobei R² ein verzweigter Kohlenwasserstoffrest ist, welcher wiederum aus zwei gleichen oder verschiedenen einwertigen aliphatischen oder aromatischen, gesättigten oder ungesättigten Kohlenwasserstoffresten mit je 12 bis 40 C-Atomen, bevorzugt 14 bis 30, besonders bevorzugt mit 16 bis 24 Kohlenstoffatomen besteht, und Aⁿ⁻ und Kat.^{m+} sowie die Anbindung des Restes R² an die hydrophile Kopfgruppe wie zuvor beschrieben.

Im Rahmen der vorliegenden Erfindung leiten sich die Reste R² bevorzugt von verzweigten primären und/oder sekundären Alkoholen ab. Hierbei sind insbesondere Guerbetalkohole, sprich durch Guerbet-Kondenstation gebildete verzweigte Alkohole, sowie durch Paraffinoxidation nach dem Bashkirov-Verfahren gebildete verzweigte sekundäre Alkohole bevorzugt. Bevorzugt sind in diesem Zusammenhang auch Alkoxylate, bevorzugt Ethoxylate und Propoxylate der zuvor genannten Alkohole, bevorzugt mit nicht mehr als 10, bevorzugt mit nicht mehr als 7, mehr bevorzugt mit nicht mehr bevorzugt als 5, noch mehr bevorzugt mit nicht mehr als 3 Alkoxyeinheiten.
Insbesondere ist es in diesem Zusammenhang bevorzugt, wenn die zweischwänzigen, langkettigen Aniontenside der allgemeinen Formel (III) oder (IV) entsprechen: 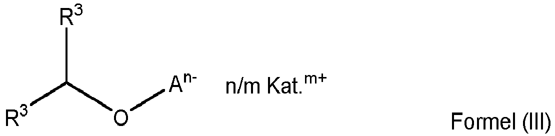 bzw. 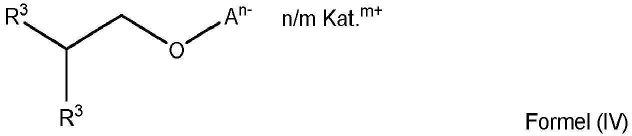 wobei die Reste R³ unabhängig voneinander gleiche oder verschiedene einwertige aliphatische oder aromatische, gesättigte oder ungesättigte Kohlenwasserstoffreste mit 12 bis 40 C-Atomen, bevorzugt 14 bis 30, besonders bevorzugt mit 16 bis 24 Kohlenstoffatomen sind, und Aⁿ⁻ und Kat.^{m+} wie zuvor beschrieben.

Im Rahmen der vorliegenden Erfindung sind insbesondere solche zweischwänzige, langkettige Aniontenside bevorzugt, welche ausgewählt sind aus den Gruppen der Dialkyl-Sulfosuccinate, der Dialkyl-Phosphate, der Dialkyl-Sulfosuccinamate, sowie der Dialkyl-Ester Tri- oder höherfunktionaler Carbonsäuren. Besonders bevorzugt sind in diesem Zusammenhang Dipalmityl-Sulfosuccinate, Distearyl-Sulfosuccinate, Stearyl-Palmityl-Sulfosuccinate, Dipalmityl-Phosphate, Distearyl-Phosphate, Stearyl-Palmityl-Phosphate, Dipalmityl-Sulfosuccinamate, Distearyl-Sulfosuccinamate, Stearyl-Palmityl-Sulfosuccinamate, Dipalmityl-Ester von Tri- oder höherfunktionaler Carbonsäuren, Distearyl-Ester von Tri- oder höherfunktionaler Carbonsäuren, Stearyl-Palmityl-Ester von Tri- oder höherfunktionaler Carbonsäuren sowie Mischungen dieser Substanzen. Weiterhin sind im Rahmen der vorliegenden Erfindung solche zweischwänzigen, langkettigen Aniontenside bevorzugt, welche ausgewählt sind aus der Gruppe der Monoalkyl-Sulfosuccinate, Monoester von Phosphorsäure, Schwefelsäure sowie Di- oder höherwertigen Carbonsäuren mit langkettigen verzweigten Alkoholen, wie zuvor beschrieben.

Die im Rahmen der vorliegenden Erfindung bevorzugten langkettigen Dialkyl-Sulfosuccinate bzw. Monoalkyl-Sulfosuccinate basierend auf verzweigten langkettigen Alkoholen sind beispielsweise synthetisch zugänglich durch Umsetzung von Maleinsäureanhydrid mit entsprechenden Alkoholen, wie zuvor beschrieben, und anschließender Sulfonierung mit Natriumhydrogensulfit.

Die im Rahmen der vorliegenden Erfindung bevorzugten Dialkyl-Sulfosuccinamate sind beispielsweise synthetisch zugänglich durch Umsetzung von Maleinsäureanhydrid mit langkettigen Aminen, wie zuvor beschrieben, und anschließend Sulfonierung mit Natriumhydrogensulfit.

Die im Rahmen der vorliegenden Erfindung bevorzugten langkettigen Dialkyl-Phosphate, sowie Mono-Phosphorsäureester verzweigter langkettiger Alkohole sind beispielsweise zugänglich durch Umsetzung von Phosphorsäure-Anhydrid (P₄O₁₀) mit entsprechenden Alkoholen, wie zuvor beschrieben, und anschließender Neutralisation des gebildeten Phosphorsäure-Partialssters.

Die im Rahmen der vorliegenden Erfindung bevorzugten Dialkyl-Ester Tri- oder höherfunktionaler Carbonsäuren, sowie Monoester von Di- oder höherfunktionaler Carbonsäuren mit verzweigten langkettigen Alkoholen sind beispielsweise synthetisch zugänglich durch Kondensation entsprechender Carbonsäuren mit entsprechenden Alkoholen, wie zuvor beschrieben, und anschließender Neutralisation der gebildeten Partialester.

Die im Rahmen der vorliegenden Erfindung bevorzugten Mono-Schwefelsäure-Ester verzweigter langkettiger Alkohole sind beispielsweise zugänglich durch Umsetzung von Schwefeltrioxid mit entsprechenden Alkoholen, wie zuvor beschrieben, und anschließender Neutralisation des gebildeten Schwefelsäure-Partialesters.

Der Begriff der Neutralisation deckt im Sinne der gesamten vorliegenden Erfindung auch eine partielle Neutralisation ab. Zur Neutralisation, umfassend partielle Neutralisation, können übliche Basen eingesetzt werden. Hierzu gehören die wasserlöslichen Metallhydroxide, wie z. B. Barium-, Strontium-, Calciumhydroxid und vorzugsweise die Hydroxide der Alkalimetalle, die in wässrigen Lösungen in freie Metall- und Hydroxid-Ionen dissoziieren, insbesondere NaOH und KOH. Hierzu gehören auch die Anhydrobasen, die mit Wasser unter Bildung von Hydroxid-Ionen reagieren, wie z.B. Bariumoxid, Strontiumoxid, Calciumoxid, Lithiumoxid, Silberoxid und Ammoniak. Neben diesen vorgenannten Alkalien sind auch solche festen Stoffe als Basen einsetzbar, die bei der Auflösung in Wasser ebenfalls alkalisch reagieren, ohne (in der festen Verbindung) HO- zu besitzen, hierzu zählen z.B. Amine wie Mono-, Di- und Trialkylamine, wobei es sich auch um funktionalisierte Alkylreste wie beispielsweise bei Amidaminen, Mono-, Di- und Trialkanolamine oder Mono-, Di- und Triaminoalkylamine handeln kann, und z. B. die Salze schwacher Säuren wie Kaliumcarbonat, Natriumcarbonat, Trinatriumphosphat usw. Des Weiteren können auch höherwertige Amine, wie z.B. Ethylendiamin, Diethylentriamin oder Triethylentetraamin zur Neutralisation verwendet werden.

Im Rahmen der vorliegenden Erfindung ist es weiterhin bevorzugt, wenn in Formel (I) - (IV) das Kation Kat.^{m+} ein Metall-Kationen, besonders bevorzugt ein Lithium-, Natrium-, Kalium-, Calcium-, Strontium-, Barium-, Barium- oder Silber-Kationen ist, wobei Natrium oder Kalium-Kationen ganz besonders bevorzugt sind. Weiterhin ist es bevorzugt, wenn es sich bei dem Kation um ein Ammonium-Kation einer protonierten Amin-Verbindung handelt, wobei Ammonium-Ionen auf Basis von Ammoniak, Aminen, wie Mono-, Di- und Trialkylamine, wobei es sich auch um funktionalisierte Alkylreste wie beispielsweise bei Amidaminen, Mono-, Di- und Trialkanolamine oder Mono-, Di- und Triaminoalkylamine handeln kann, sowie höherwertigen Aminen, wie z.B. Ethylendiamin, Diethylentriamin oder Triethylentetraamin besonders bevorzugt sind.

Wie zuvor beschrieben können die zweischwänzigen, langkettigen Aniontenside im Sinne der vorliegenden Erfindung durch Neutralisation von Partialestern höherwertiger Säuren, z.B. langkettige Phosphorsäure-Partialester, erhalten werden. Da im Rahmen der vorliegenden Erfindung der Begriff der Neutralisation auch eine partielle Neutralisation erfasst, kann es sich bei dem Kation K in diesem Fall auch um ein an die Kopfgruppe gebundenes Wasserstoff-Atom bzw. ein dissoziiertes Hydroniumlon handeln.

Wie bereits beschrieben, sieht die vorliegende Erfindung die Verwendung von langkettigen, zweischwänzigen Aniontensiden, wie zuvor beschrieben, als Additive in wässrigen Polymerdispersionen, bevorzugt in wässrigen Polyurethandispersionen vor. Die Polymer-Dispersionen sind hierbei bevorzugt ausgewählt aus der Gruppe wässriger Polystyrol-Dispersionen, Polybutadien-Dispersionen, Poly(meth)acrylat-Disperionen, Polyvinylester-Dispersionen und/oder Polyurethan-Dispersionen. Der Festkörper-Anteil dieser Dispersionen liegt bevorzugt im Bereich von 20 - 70 Gew.-%, mehr bevorzugt im Bereich von 25 - 65 Gew.-%. Erfindungsgemäß besonders bevorzugt ist die Verwendung von langkettigen, zweischwänzigen Aniontensiden als Additive in wässrigen Polyurethan-Dispersionen. Insbesondere bevorzugt sind hierbei Polyurethan-Dispersionen basierend auf Polyester-, Polyesteramid-, Polycarbonat-, Polyacetal- und/oder Polyether-Polyolen.

Im Rahmen der vorliegenden Erfindung ist es bevorzugt, wenn die Konzentration der zweischwänzigen, langkettigen Aniontensiden bezogen auf das Gesamtgewicht der wässrigen Polymerdispersion im Bereich von 0,1 - 20 Gew.-%, besonders bevorzugt im Bereich von 0,2 - 15 Gew.-%, insbesondere bevorzugt im Bereich von 0,5 - 10 Gew.-% liegt.

Bevorzugt werden die zweischwänzigen, langkettigen Aniontensiden in wässrigen Polymer-Dispersionen als Schaumhilfsmittel bzw. Schaumstabilisatoren zum Aufschäumen der Dispersionen verwendet, d.h. als Schaumadditive. Weiterhin können sie jedoch auch als Trocknungshilfsmittel, Verlaufsadditive, Netz-Mittel sowie Rheologieadditive verwendet werden, was ebenfalls bevorzugten Ausführungsformen der vorliegenden Erfindung entspricht.

Neben den erfindungsgemäßen zweischwänzigen, langkettigen Aniontensiden können die wässrigen Polymerdispersionen noch weitere Zusätze/Formulierungskomponenten wie Farb-Pigmente, Füllstoffe Mattierungsmittel, Stabilisatoren, wie Hydrolyse- oder UV-Stabilisatoren, Antioxidantien, Absorber, Vernetzer, Verlaufsadditive, Verdicker oder sowie weitere Co-Tenside enthalten.

Die zweischwänzigen, langkettigen Aniontensiden können der wässrigen Dispersion sowohl pur als auch abgemischt in einem geeigneten Lösemittel zugeben werden. Bevorzugte Lösemittel sind in diesem Zusammenhang ausgewählt aus Wasser, Propylenglykol, Dipropylenglykol, Polypropylenglykol, Butyldiglykol, Butyltriglykol, Ethylenglykol, Diethylenglykol, Polyethylenglykol, Polyalkylenglykole auf Basis von EO, PO, BO und/oder SO, Alkoholalkoxylate auf Basis von EO, PO, BO und/oder SO sowie Mischungen dieser Substanzen, wobei wässrige Verdünnungen bzw. Abmischungen ganz besonders bevorzugt sind. In Abmischungen bzw. Verdünnungen der zweischwänzigen, langkettigen Aniontenside sind mindestens 5 Gew.-%, mehr bevorzugt mindestens 10 Gew.-%, noch mehr bevorzugt mindestens 15 Gew.-% der zweischwänzigen, langkettigen Aniontenside enthalten.

Im Fall von wässrigen Verdünnungen bzw. Abmischungen der erfindungsgemäßen zweischwänzigen, langkettigen Aniontenside kann es vorteilhaft sein, wenn zur Verbesserung der Formulierungseigenschaften (Viskosität, Homogenität etc.) der Abmischung hydrotrope Verbindungen zugesetzt werden. Hydrotrope Verbindungen sind hierbei wasserlösliche organische Verbindungen, welche aus einem hydrophilen und einem hydrophoben Part bestehen, jedoch zu niedermolekularsind, um tensidische Eigenschaften aufzuweisen. Sie führen zu einer Verbesserung der Löslichkeit bzw. der Löslichkeitseigenschaften organischer, insbesondere hydrophober organischer Substanzen in wässrigen Formulierungen. Der Begriff hydrotrope Verbindungen ist dem Fachmann bekannt. Als hydrotrope Verbindungen sind im Rahmen der vorliegenden Erfindung Alkali- und Ammonium-Toluolsulfonate, Alkali- und Ammonium-Xylolsulfonate, Alkali- und Ammonium-Naphtalinsulfonate, Alkali- und Ammonium-Cumolsulfonate sowie Phenol-Alkoxylate, insbesondere Phenol-Ethoxylate mit bis zu 6 Alkoxylat-Einheiten bevorzugt.

Weiterhin kann es vorteilhaft sein, dass die zweischwänzigen, langkettigen Aniontenside nicht pur, sondern in Kombination mit weiteren Co-Tensiden als Additive in wässrigen Polymerdispersionen, bevorzugt in wässrigen Polyurethan-Dispersionen verwendet werden. Diese können z.B. für eine verbesserte Systemverträglichkeit oder, im Fall von vorformulierten Tensidmischungen für verbesserte Formulierungseigenschaften eingesetzt werden. Erfindungsgemäß bevorzugte Co-Tenside sind in diesem Zusammenhang z.B. freie Fettalkohole, Fettsäureamide, Ethylenoxid-Propylenoxid-Blockcopolymere, Betaine, wie z.B. Amido-Propyl-Betaine, Aminoxide, quaternäre Ammonium-Tensid, Amphoacetate Ammonium und/oder Alkali-Salze von Fettsäure, Alkylsulfate, Alkylethersulfate, Alkylsulfonate, Alkylbenzolsulfonate, Alkylphosphate, Alkyl-Sulfosuccinate, Alkyl-Sulfosuccinamate und Alkylsarcosinate sowie Mischungen dieser Substanzen, wobei freie Fettalkohole, bevorzugt mit 12 bis 40, mehr bevorzugt mit 14 - 30, noch mehr bevorzugt mit 16 - 24 Kohlenstoffatomen, sowie Alkylsulfate mit 12 bis 40, mehr bevorzugt mit 14 - 30, noch mehr bevorzugt mit 16 - 24, sowie Mischungen dieser Substanzen ganz besonders bevorzugt sind. Weiterhin kann es sich bei dem Co-Tensid um silikonbasierte Tenside, wie z.B. Trisiloxan-Tenside oder Polyether-Siloxane handeln. Im Falle von Ammonium und/oder Alkali-Salzen von Fettsäure ist es bevorzugt, wenn diese weniger als 25 Gew.-% Stearat-Salze enthalten, insbesondere frei von Stearat-Salzen sind.

Im Falle von Kombinationen der erfindungsgemäßen zweischwänzigen, langkettigen Aniontenside mit weiteren Co-Tensiden, wie zuvor beschrieben, ist es insbesondere bevorzugt, wenn diese Kombinationen zwischen 1 und 60 Gew.-%, bevorzugt zwischen 2 und 50 Gew.-%, mehr bevorzugt zwischen 3 und 40 Gew.-%, noch mehr bevorzugt zwischen 5 und 30 Gew.-% Co-Tensid, bezogen auf die Kombination aus erfindungsgemäßen zweischwänzigen, langkettigen Aniontenside und Co-Tensid, aufweisen.

Da, wie oben beschrieben, die erfindungsgemäße Verwendung von zweischwänzigen, langkettigen Aniontensiden zu einer deutlichen Verbesserung von aus wässrigen Polymerdispersionen hergestellten, porösen Polymerbeschichtungen führt, sind wässrige Polymerdispersionen enthaltend mindestens eines der erfindungsgemäßen zweischwänzigen, langkettigen Aniontenside wie zuvor ausführlich beschrieben, ebenfalls Gegenstand der vorliegenden Erfindung.

Noch ein Gegenstand der vorliegenden Erfindung sind aus wässrigen Polymerdispersionen hergestellte poröse Polymerschichten, erhalten unter der erfindungsgemäßen Verwendung von zweischwänzigen, langkettigen Aniontensiden, wie zuvor ausführlich beschrieben.

Bevorzugt lassen sich die erfindungsgemäßen porösen Polymerbeschichtungen durch ein Verfahren herstellen umfassend die Schritte
a) Bereitstellen einer Mischung enthaltend mindestens eine wässrige Polymerdispersion, mindestens ein erfindungsgemäßes zweischwänziges, langkettiges Aniontensid, sowie ggf. weitere Additive
b) Aufschäumen der Mischung zu einem Schaum
c) optional Zugabe mindestens eines Verdickers zur Einstellung der Viskosität des Nassschaums
d) Auftragen einer Beschichtung der geschäumten Polymer-Dispersion auf einen geeigneten Träger
e) Trocknen / Aushärten der Beschichtung.

Die porösen Polymerbeschichtungen weisen Poren vorzugsweise im Mikrometerbereich auf, bevorzugt mit einer mittleren Zellgröße kleiner 350 µm, weiter bevorzugt kleiner 200 µm, insbesondere bevorzugt kleiner 150 µm, ganz besonders bevorzugt kleiner 100 µm. Die mittlere Zellgröße lässt sich vorzugsweise mikroskopisch, vorzugsweise durch Elektronenmikroskopie bestimmen. Hierfür wird ein Querschnitt der porösen Polymerbeschichtung mittels eines Mikroskops mit einer ausreichenden Vergrößerung betrachtet und die Größe von mindestens 25 Zellen ermittelt. Die mittlere Zellgröße ergibt sich dann als arithmetisches Mittel der betrachteten Zellen bzw. Zellgrößen.

Mit Blick auf bevorzugte Ausgestaltungen, insbesondere mit Blick auf die in dem Verfahren bevorzugt einsetzbaren langkettigen, zweischwänzigen Aniontenside und Polymerdispersionen sei auf die vorangegangene Beschreibung und auch auf die zuvor genannten bevorzugten Ausführungsformen, insbesondere wie in den Ansprüchen dargestellt, verwiesen.

Es wird klargestellt, dass die Verfahrensschritte des erfindungsgemäßen Verfahrens wie oben dargestellt keiner festen zeitlichen Abfolge unterworfen sind. So kann beispielsweise der Verfahrensschritt c) bereits zeitgleich mit dem Verfahrensschritt a) ausgeführt werden.

Es entspricht einer bevorzugten Ausführungsform der vorliegenden Erfindung, wenn im Verfahrensschritt b) die wässrige Polymerdispersion durch das Aufbringen hoher Scherkräfte aufgeschäumt wird. Das Aufschäumen kann hierbei unter zu Hilfenahme von dem Fachmann geläufigen Scheraggregaten wie z.B. Dispermaten, Dissolvern, Hansa-Mixern oder Oakes-Mixern erfolgen.

Weiterhin ist er bevorzugt, wenn der am Ende des Verfahrensschritt c) hergestellte Nassschaum eine Viskosität von mindestens 5, bevorzugt von mindestens 10, mehr bevorzugt von mindestens 15 noch mehr bevorzugt von mindestens 20 Pa·s, jedoch von maximal 500 Pa s, bevorzugt von maximal 300 P as, mehr bevorzugt von maximal 200 Pa s noch mehr bevorzugt von maximal 100 Pa s aufweist. Die Viskosität des Schaums lässt sich hierbei vorzugsweise mit Hilfe eines Viskosimeters der Firma Brookfield, Modell LVTD ausgerüstet mit einer Spindel LV-4, bestimmen. Entsprechende Messmethoden zur Bestimmung der Nassschaumviskosität sind dem Fachmann bekannt.

Es entspricht einer bevorzugten Ausführungsform der vorliegenden Erfindung, wenn im Verfahrensschritt b) der Schaum möglichst homogen und feinzellig ist. Der Fachmann kann das gewünschtenfalls auf übliche Weise durch einfache direkte Sichtprüfung mit bloßem Auge oder mit optischen Hilfsmitteln, wie z.B. Lupen, Mikroskopen, anhand seiner üblichen Erfahrungswerte überprüfen. "Feinzellig" bezieht sich auf die Zellgröße. Je kleiner die mittlere Zellgröße, desto feinzelliger der Schaum. Gewünschtenfalls kann die Feinzelligkeit zum Beispiel mit einem Lichtmikroskop oder mit einem Rasterelektronenmikroskop bestimmt werden. "Homogen" meint die Zellgrößenverteilung. Ein homogener Schaum weißt eine möglichst enge Zellgrößenverteilung auf, so dass alle Zellen in etwa gleich groß sind. Quantifizieren könnte man dies wiederum mit einem Lichtmikroskop oder mit einem Rasterelektronenmikroskop.

Wie oben bereits beschrieben, können dem System zur Einstellung der Nassschaumviskosität zusätzliche Verdicker zugesetzt werden.

Bevorzugt sind die Verdicker, die im Rahmen der Erfindung vorteilhafterweise eingesetzt werden können, hierbei ausgewählt aus der Klasse der Assoziativ-Verdicker. Assoziativverdicker sind hierbei Substanzen, die durch Assoziation an den Oberflächen der in den Polymerdispersionen enthaltenen Partikeln zu einem verdickenden Effekt führen. Der Begriff ist dem Fachmann bekannt. Bevorzugte Assoziativverdicker sind hierbei ausgewählt aus Polyurethanverdickern, hydrophob-modifizierten Polyacrylat-Verdickern, hydrophob-modifizierten Polyether-Verdickern sowie hydrophob modifizierten Celluloseethern. Ganz besonders bevorzugt sind Polyurethanverdicker. Weiterhin ist es im Rahmen der vorliegenden Erfindung bevorzugt, wenn die Konzentration der Verdicker bezogen auf die Gesamtzusammensetzung der Dispersion im Bereich von 0,01 - 10 Gew.-%, mehr bevorzugt im Bereich von 0,05 - 5 Gew.-%, ganz besonders bevorzugt im Bereich von 0,1 - 3 Gew.-% liegt.

Im Rahmen der vorliegenden Erfindung ist es zudem bevorzugt, wenn in Verfahrensschritt d) Beschichtungen der geschäumten Polymerdispersion mit einer Schichtdicke von 10 - 10000 µm, bevorzugt von 50 - 5000 µm, mehr bevorzugt von 75 - 3000 µm noch mehr bevorzugt von 100 - 2500 µm hergestellt werden. Beschichtungen der geschäumten Polymerdispersion können durch dem Fachmann geläufige Methoden, wie z.B. Aufrakeln, hergestellt werden. Hierbei können sowohl direkte also auch indirekte Beschichtungsprozesse (sogenanntes Transfer-Coating) verwendet werden.

Außerdem ist es ihm Rahmen der vorliegenden Erfindung bevorzugt, wenn in Verfahrensschritt e) die Trocknung der geschäumten und beschichteten Polymerdispersion bei erhöhten Temperaturen erfolgt. Erfindungsgemäß bevorzugt sind hierbei Trocknungstemperaturen von min. 50 °C, bevorzugt von 60 °C, mehr bevorzugt von mindestens 70 °C. Weiterhin ist es möglich die geschäumten und beschichteten Polymerdispersionen mehrstufig bei verschiedenen Temperaturen zu trocknen, um das Auftreten von Trocknungsdefekten zu vermeiden. Entsprechende Trocknungstechniken sind in der Industrie verbreitet und dem Fachmann bekannt.

Wie bereits beschrieben können die Verfahrensschritte c) - e) unter Zuhilfenahme von verbreiteten, dem Fachmann bekannten Methoden erfolgen. Eine Übersicht hierrüber ist beispielsweise in "Coated and laminated Textiles" (Walter Fung, CR-Press, 2002) gegeben.

Im Rahmen der vorliegenden Erfindung sind insbesondere solche porösen Polymerbeschichtungen enthaltend zweischwänzige, langkettige Anionen-Tenside bevorzugt, welche eine mittlere Zellgröße kleiner 350 µm, bevorzugt kleiner 200 µm, insbesondere bevorzugt kleiner 150 µm, ganz besonders bevorzugt kleiner 100 µm aufweisen. Die mittlere Zellgröße lässt sich vorzugsweise mikroskopisch, vorzugsweise durch Elektronenmikroskopie bestimmen. Hierfür wird ein Querschnitt der porösen Polymerbeschichtung mittels eines Mikroskops mit einer ausreichenden Vergrößerung betrachtet und die Größe von mindestens 25 Zellen ermittelt. Um eine ausreichende Statistik dieser Auswertemethode zu erhalten sollte die Vergrößerung des Mikroskops vorzugsweise so gewählt sein, dass sich mindestens 10x10 Zellen im Beobachtungsfeld befinden. Die mittlere Zellgröße ergibt sich dann als arithmetisches Mittel der betrachteten Zellen bzw. Zellgrößen. Diese Zellgrößenbestimmung mittels Mikroskopie ist dem Fachmann geläufig.

Die erfindungsgemäßen porösen Polymerschichten (bzw. Polymerbeschichtungen) enthaltend mindestens eines der erfindungsgemäßen zweischwänzigen, langkettigen Aniontenside und ggf. weitere Additive können beispielsweise in der Textil-Industrie, z.B. für Kunstleder-Materialien, in der Bauindustrie, in der Elektronik-Industrie, in der Sportindustrie oder in der Automobil-Industrie verwendet werden. So können auf Basis der erfindungsgemäßen porösen Polymerbeschichtungen Gebrauchsgegenstände wie Schuhe, Einlegesohlen, Taschen, Koffer, Etuis, Kleidung, Automobilteile, bevorzugt Sitzbezüge, Bespannungen von Türteilen, Armaturenbrettteilen, Lenkrädern und/oder Griffen sowie Schaltsäcken, Einrichtungsgegenstände wie Schreibunterlagen, Kissen oder Sitzmöbeln, Gap Filler in elektronischen Geräten, Polster- und Dämpfungsmaterialien in medizinischen Anwendungen oder Klebebänder hergestellt werden.

### Beispiele

### Substanzen:

SYNTEGRA^{®} YS:3000: MDI (Methyl Diphenyl Diisocyanate) basierte Polyurethandispersion von der Firma DOW. Das Produkt enthält bedingt durch seinen Herstellprozess 1 - 3 Gew.- % des anionischen Co-Tensids Natriumdodecylbenzolsulfonat (CAS: 25155-30-0).
IMPRANIL^{®} DLU: aliphatische Polycarbonatester-Polyether Polyurethandispersion von der Firma Covestro
REGEL^{®} WX 151: wässrige Polyurethandispersion von der Firma Cromogenia
CROMELASTIC^{®} PC 287 PRG: wässrige Polyurethandispersion von der Firma Cromogenia
STOKAL^{®} STA: Ammonium-Stearat (ca. 30 %ig in H₂O) von der Firma Bozetto
STOKAL^{®} SR: Talkfett-basiertes Natrium-Sulfosuccinamat (ca. 35 %ig in H₂O) von der Firma Bozetto
   Natriumdodecylbenzolsulfonat (LAS; CAS: 25155-30-0) wurde von Sigma Aldrich bezogen. Dieses ist ein gängiges zur Herstellung von wässrigen Polyurethan-Dispersionen verwendetes Co-Tensid.
ECO Pigment Black: Wässrige Pigment-Dispersion (schwarz) von der Firma Cromogenia.
TEGOWET^{®} 250: Polyether-Siloxan-basiertes Verlaufsaddive von der Firma Evonik Industries AG
ORTEGOL^{®} PV 301: Polyurethan-basierter Assoziativ-Verdicker der Firma Evonik Industries AG.
REGEL^{®} TH 27: Isocyanat-basiertes Vernetzungsadditiv von der Firma Cromogenia

### Viskositätsmessungen:

Alle Viskositätsmessungen wurden mit einem Viskosimeter der Firma Brookfield, Typ LVTD ausgerüstet mit Spindel LV-4, bei einer konstanten Rotationsgeschwindigkeit von 12 rpm durchgeführt. Für die Viskositätsmessungen wurden die Proben in ein 100 ml Glas gefüllt, in welches die Messspindel eintauchte. Es wurde stets gewartet, bis das Viskosimeter einen konstanten Messwert anzeigte.

### Methode zur Säurezahlbestimmung:

Geeignete Methoden zur Bestimmung der Säurezahl sind insbesondere solche gemäß DGF C-V 2, DIN EN ISO 2114, Ph.Eur. 2.5.1, ISO 3682 und ASTM D 974.

### Beispiel 1: Synthese von Phosphorsäurestearvlester:

Stearylalkohol (≥95 %, 178.7 g, 0.661 mol) wurde unter Rühren und N₂-Einleitung auf 70 °C erhitzt und anschließend wurde P₄O₁₀ (21.31 g, 0.0751 mol) in kleinen Portionen zugegeben, so dass die Temperatur des Reaktionsgemisches nicht über 80 °C stieg. Nach Beendigung der Zugabe des P₄O₁₀ wurde das Reaktionsgemisch für 3 h bei 80 °C unter N₂-Einleitung gerührt, bis die Säurezahl kostant war. Nach dem Ausgießen und Abkühlen wurde ein farbloser Feststoff mit einer Säurezahl von 127 mg KOH/g erhalten.

### Beispiel 2: Synthese von Citronensäurestearvlester:

Stearylalkohol (≥95 %, 275.2 g, 1.02 mol, 2.1 Äq.) wurde unter Rühren und N₂-Einleitung auf 70 °C erhitzt und anschließend wurde Citronensäure (wasserfrei, 93.10 g, 0.485 mol, 1.0 Äq.) zugegeben. Das Reaktionsgemisch wurde auf 140 °C erhitzt und bei einem Druck von 150 mbar für 3 h unter N₂-Einleitung gerührt, bis eine Säurezahl von 62 mg KOH/g erreicht war. Das Gemisch wurde bei einer Temperatur von 120 °C über eine Druckfilterpresse (1 µm) bei 2 bar filtriert. Nach dem Abkühlen wurde ein farbloser Feststoff mit einer Säurezahl von 57 mg KOH/g erhalten.

### Beispiel 3: Verqleichsbeispiel:

Neben den erfindungsgemäßen Tensiden wurde noch eine Vergleichstensid basierend auf Polyglycerin-3-Stearat verwendet, welches hergestellt wurde durch Umsetzung von 103.3 g Polyglycerin - OHZ = 1124 mg KOH/g, Mw = 240 g/mol - mit 155.0 g technischer Stearinsäure.

### Beispiel 4: Abmischung der Tenside:

Die erfindungsgemäßen Tenside aus Bespiel 1 und 2 sowie das Polyglycerinester-basierte Vergleichstensid aus Beispiel 3 wurden gemäß der in Tabelle 1 aufgeführten Zusammensetzungen abgemischt und anschließend bei 80 °C homogenisiert. Die erfindungsgemäßen Tensidformulierungen 1 und 2 wurden nach dem Abmischen mit KOH auf pH = 7 neutralisiert. Das Vergleichstensid 3 wies nach Abmischung bereits einen pH-Wert von 7 auf und wurde nicht neutralisiert.

**Tabelle 1: Zusammensetzung von im folgenden verwendeten Tensidabmischungen:**

| | **Tensid 1** | **Tensid 2** | **Tensid 3** |
|---|---|---|---|
| Phosphorsäurestearylester (aus Beispiel 1) | 20,0 g | | --- |
| Citronensäurestearylester (aus Bespiel 2) | | 20,0 g | |
| Polyglycerin-3-Stearat (Vergleichs beispiel) | | | 20,0 g |
| Stearylalkohol | 4g | 4g | 4 g |
| Wasser | 76,0 g | 76,0 g | 76,0 g |

### Beispiel 5: Aufschäumversuche:

Zur Ausprüfung der Wirksamkeit der erfindungsgemäßen Additiv-Kombination wurde eine Reihe an Verschäumungsexperimente durchgeführt. Hierfür wurde in einem ersten Schritt die Polyurethan-Dispersion IMPRANIL^{®} DLU von der Firma Covestro verwendet. Als Schaumstabilisator wurden die erfindungsgemäßen Tensidformulierungen 1 und 2 (siehe Tabelle 1) sowie eine Kombination aus den beiden Tensiden Stokal STA (Ammoniumstearat) und Stokal SR (Natrium-Sulfosuccinamat) als Vergleich verwendet. Tabelle 2 gibt einen Überblick über die Zusammensetzungen der jeweiligen Versuche.

Alle Verschäumungsexperimente wurden in Handversuchen durchgeführte. Hierfür wurden Polyurethandispersion und Tensid zunächst in einem 500 ml Plastikbecher vorgelegt und für 3 min. bei 1000 rpm mit einem Disslover, ausgerüstet mit einer Dispergierscheibe (Durchmesser = 6 cm), homogenisiert. Zum Aufschäumen der Mischungen wurde die Schergeschwindigkeit anschließend auf 2000 rpm erhöht, wobei darauf geachtet wurde, dass die Dispergierscheibe stets soweit in die Dispersion eintauchte, dass sich eine ordentliche Trombe bildete. Bei dieser Geschwindigkeit wurden die Mischungen auf ein Volumen von ca. 425 ml geschäumt. Anschließend wurde die Mischung für weitere 15 Minuten bei 1000 rpm geschert. Die Dispergierscheibe wurde in diesem Schritt so tief in die Mischungen eingetaucht, dass keine weitere Luft ins System eingetragen wurde, jedoch das komplette Volumen noch in Bewegung war.

**Tabelle 2: Übersicht Schaumformulierungen:**

| | **#1** | **#2** | **#3** |
|---|---|---|---|
| IMPRANIL^{®} DLU | 150 g | 150 g | 150 g |
| Tensid 1 | 4 g | - | - |
| Tensid 2 | - | 4 g | - |
| Stokal STA | - | - | 2 g |
| Stokal SR | - | - | 2 g |
| Viskosität Nassschaum [mPa s] | 7100 | 7400 | 4000 |

In allen Fällen wurden am Ende dieses Aufschäumvorgangs feine, homogene Schäume erhalten. Auffällig war, dass die Schäume #1 und #2, welche mit den erfindungsgemäßen Tensiden 1 und 2 hergestellt wurden, eine höhere Viskosität aufwiesen (siehe Tabelle 2). Die Schäume wurde mit Hilfe eines mit einem Kastenrakel (Rakelstärke = 800 µm) ausgerüsteten Filmziehgeräts (Typ AB3220 der Firma TQC) auf eine silikonisierte Polyesterfolie aufgebracht und anschließend für 5 min. bei 60 °C sowie für weitere 5 min bei 120 °C getrocknet.

Verglichen mit der so erhaltenen Probe #3 zeichneten sich die getrockneten erfindungsgemäßen Proben #1 und #2 durch eine homogenere makroskopische Erscheinung sowie durch einen samtigeren Griff aus. In elektronenmikroskopischen Untersuchungen konnte zu dem eine feinzelligere Porenstruktur festgestellt werden.

### Beispiel 6: Migrationsversuche:

Zur Bewertung der Oberflächenmigration der erfindungsgemäßen Tenside wurden Kunstledermaterialien nach folgendem Verfahren herstellt. Zunächst wurde eine Decklackbeschichtung auf eine silikonisierte Polyesterfolie aufgebracht (Schichtstärke 100 µm). Diese wurde anschließend bei 100 °C für 3 Minuten getrocknet. Anschließend wurde eine Schaumschicht auf die getrocknete Decklackschicht beschichtet (Schichtstärke 800 µm) und für 5 Minuten bei 60 °C und 5 Minuten bei 120 °C getrocknet. Auf den getrockneten Schaumschicht wurde in einem letzten Schritt eine wässrige Klebstoff-Schicht (Schichtstärke 100 µm) gestrichen und anschließend ein textiler Träger auf die noch feuchte Klebstoff-Schicht laminiert. Das fertige Laminat wurde erneut bei 5 Minuten bei 120 °C getrocknet und anschließend von der Polyesterfolie abgelöst.

Alle Beschichtungs- und Trocknungsvorgänge wurden hierbei mit einem *Labcoater LTE-S* von der Firma *Mathis AG* durchgeführt. Decklack- und Klebstoff-Schicht wurden hierbei entsprechend der in Tabelle 3 aufgelisteten Zusammensetzungen formuliert, als Schaumschicht wurden die in Tabelle 2 aufgeführten Schaumformulierungen verwendet, welche nach dem in Beispiel 5 beschriebenen Verfahren aufgeschäumt wurden.

Zur Bewertung der Tensidmigration wurden die Kunstlederproben nach ihrer Herstellung für 30 Minuten in 100 °C heißes Wasser platziert und anschließend über Nacht bei Raumtemperatur getrocknet. Nach dieser Behandlung wies die aus den Tensiden Stokal STA / SR hergestellte Vergleichsprobe (Schaumformulierung #3, Tabelle 2) deutlich sichtbare weiße Flecken an der Oberfläche des Kunstleders auf, während diese Oberflächenverfärbungen bei den mit den erfindungsgemäßen Tensiden hergestellten Proben (Schaumformulierung #1 und #2, Tabelle 2) nicht zu beobachten waren.

**Tabelle 3: Decklack- und Klebstoff-Formulierung zur Herstellung von Kunstledermaterialien:**

| | **Decklack** | **Klebstoff** |
|---|---|---|
| CROMELASTIC^{®} PC 287 PRG | 100 g | - |
| REGEL^{®} WX 151 | - | 100 g |
| ECO Pigment Black | 10 g | 5 g |
| TEGOWET^{®} 250 | 0,2 g | 0,2 g |
| REGEL^{®} TH 27 | 6 g | 6 g |
| ORTEGOL^{®} PV 301 | 7 g | 5 g |

### Beispiel 7: Co-Tensidverträqlichkeit:

Zur Bewertung der Co-Tensidverträglichkeit wurden weitere Aufschäumversuche mit dem PUD-System SYNTEGRA^{®} YS:3000 durchgeführt. Dieses enthält 1 - 3 Gew.- % des anionischen Co-Tensids Natriumdodecylbenzolsulfonat (CAS: 25155-30-0). Als Tenside in diesen Versuchen wurden die in Tabelle 1 aufgeführten Tensidformulierungen 1-3 verwendet. Tabelle 4 gibt einen Überblick über die Zusammensetzung der Schaumformulierungen.

**Tabelle 4: Übersicht Schaumformulierungen:**

| | **#4** | **#5** | **#6** |
|---|---|---|---|
| SYNTEGRA^{®} YS 3000 | 150 g | 150 g | 150 g |
| Tensid 1 | 4 g | - | - |
| Tensid 2 | - | 4 g | - |
| Tensid 3 | - | - | 4 g |

Auf Basis dieser Formulierungen wurden nach dem in Beispiel 5 beschriebenen Verfahren Schaumbeschichtungen hergestellt. Hierbei war auffällig, dass die mit dem nicht erfindungsgemäßem Tensid 3 hergestellte Probe #6 ein deutlich gröbere und inhomogenere Schaumstruktur aufwies. Außerdem konnten nach dem Trocknen der Schaumbeschichtung deutliche Risse in der Schaumstruktur beobachtet werden, was ein Hinweis auf eine unzureichende Stabilisierung des Schaums ist. Die mit den erfindungsgemäßen Tensiden hergestellten Proben #4 und #5 hingegen zeichneten sich erneut durch eine äußerst feinzellige und homogene Schaumstruktur aus. Auch waren sie frei von Trocknungsrissen.

Darüber hinaus wurde eine weitere Reihe an Verschäumungsexperimenten durchgeführt, in welcher das eigentlich Co-Tensid-freie System IMPRANIL^{®} DLU bewusst mit Natriumdodecylbenzolsulfonat, wie bereits beschrieben ein gängiges Co-Tensid zur PUD-Stabilisierung, additiviert wurde. Auch in diesen Versuchen wurden die Tabelle 1 aufgeführten Tensidformulierungen 1-3 verwendet. Tabelle 5 gibt einen Überblick über die Zusammensetzung der Schaumformulierungen.

**Tabelle 5: Übersicht Schaumformulierungen:**

| | **#7** | **#8** | **#9** |
|---|---|---|---|
| IMPRANIL^{®} DLU | 150 g | 150 g | 150 g |
| Natriumdodecylbenzolsulfonat | 1,5 g | 1,5 g | 1,5 g |
| Tensid 1 | 4 g | - | - |
| Tensid 2 | - | 4 g | - |
| Tensid 3 | - | - | 4 g |

Auch hier wurden nach dem in Beispiel 5 beschriebenen Verfahren Schaumbeschichtungen hergestellt. Hierbei fiel wiederum auf, dass die mit dem nicht erfindungsgemäßen Tensid 3 hergestellte Probe #9 Trocknungsrisse sowie eine deutlich gröbere Zellstruktur aufwies, wohingegen die beiden erfindungsgemäßen Proben #7 und #8 wieder eine feine und homogene Zellstruktur zeigten und frei von Defekten waren. Hier konnte nahezu kein Unterschied zu den analogen, cotensidfreien Proben #1 und #2 (siehe Beispiel 5) beobachtet werden. Diese Versuche belegen somit die deutlich verbesserte Co-Tensidverträglichkeit der erfindungsgemäßen Tenside.

## Patentansprüche

1. Verwendung von zweischwänzigen, langkettigen Aniontensiden als Additive, vorzugsweise als Schaumadditive, in wässrigen Polymer-Dispersionen, vorzugsweise in wässrigen Polyurethandispersionen.

2. Verwendung nach Anspruch 1, **dadurch gekennzeichnet, dass** das zweischwänzige, langkettige Aniontensid anionische hydrophile Kopfgruppe sowie zwei gleiche oder verschiedene langkettige hydrophobe Kohlenwasserstoff-Reste aufweist.

3. Verwendung nach Anspruch 1 oder 2, **dadurch gekennzeichnet, dass** das zweischwänzige, langkettige Aniontensid der allgemeinen Formel (I) entspricht: 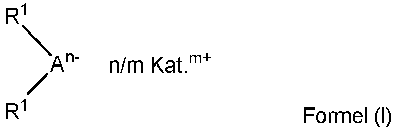 wobei die Reste R¹ unabhängig voneinander gleiche oder verschiedene einwertige gesättigte oder ungesättigte aliphatische oder aromatische Kohlenwasserstoffreste mit 12 bis 40 C-Atomen, bevorzugt 14 bis 30, besonders bevorzugt mit 16 bis 24 Kohlenstoffatomen sind, und wobei Aⁿ⁻ die anionische Kopfgruppe des Tensids ist, welche n negative Ladungen trägt, wobei n = 1 - 3, mehr bevorzugt 1 - 2, noch mehr bevorzugt gleich 1, und wobei Kat.^{m+} das kationische Gegenion ist, welches m positive Ladungen trägt, wobei m = 1 - 10, bevorzugt 1 - 5, mehr bevorzugt 1 - 2, noch mehr bevorzugt gleich 1.

4. Verwendung nach mindestens einem der Ansprüche 1 bis 3, **dadurch gekennzeichnet, dass** sich der Rest R¹ von langkettigen Alkoholen, Carbonsäuren oder Alkylaminen mit mindestens 12 bis 40 C-Atomen, bevorzugt 14 bis 30, besonders bevorzugt mit 16 bis 24 Kohlenstoffatomen ableitet.

5. Verwendung nach mindestens einem der Ansprüche 1 bis 4, **dadurch gekennzeichnet, dass** sich der Rest R¹ ableitet von Laurylalkohol (1-Dodecanol), Myristylalkohol (1-Tetradecanol), Cetylalkohol (1-Hexadecanol), Margarylalkohol (1-Heptadecanol), Stearylalkohol (1-Octadecanol), Arachidylalkohol (1-Eicosanol), Behenylalkohol (1-Docosanol), Lignocerylalkohol (1-Tetracosanol), Cerylalkohol (1-Hexacosanol), Montanylalkohol (1-Octacosanol), Melissylalkohol (1-Triacontanol), Palmitoleylalkohol (cis-9-Hexadecen-1-ol), Oleylalkohol (cis-9-Octadecen-1-ol) und/oder Elaidylalkohol (trans-9-Octadecen-1-ol) sowie Mischungen dieser Substanzen, wobei Cetylalkohol und/oder Stearylalkohol sowie Mischungen dieser beiden Substanzen besonders bevorzugt sind, und/oder ableitet von Laurinsäure (Dodecansäure), Myristinsäure (Tetradecansäure), Palmitinsäure (Hexadecansäure), Stearinsäure (Octadecansäure), Arachinsäure (Eicosansäure), Behensäure (Docosansäure), Lignocerinsäure (Tetracosansäure), Palmitölsäure ((Z)-9-Hexadecensäure), Ölsäure ((Z)-9-Hexadecensäure), Elaidinsäure ((E)-9-Octadecensäure), cis-Vaccensäure ((Z)-11-Octadecensäure), Linolsäure ((9Z,12Z)-9,12-Octadecadiensäure), alpha-Linolensäure ((9Z,12Z,15Z)-9,12,15-Octadecatriensäure), gamma-Linolensäure ((6Z,9Z,12Z)-6,9,12-Octadecatriensäure), Di-homo-gamma-Linolensäure ((8Z,11Z,14Z)-8,11,14-Eicosatriensäure), Arachidonsäure ((5Z,8Z,11Z,14Z)-5,8,11,14- Eicosatetraensäure), Erucasäure ((Z)-13-Docosensäure), Nervonsäure ((Z)-15-Tetracosensäure), Rizinolsäure, Hydroxystearinsäure und/oder Undecenylsäure, sowie deren Mischungen, wie z.B. Rapsölsäure, Soyafettsäure, Sonnenblumenfettsäure, Erdnussfettsäure und/oder Tallölfettsäure, wobei Palmitinsäure und/oder Stearinsäure sowie Mischungen dieser Substanzen besonders bevorzugt sind, und/oder ableitet von Laurylamin (1-Dodecylamin), Myristylamin (1-Tetradecylamin), Cetylamin (1-Hexadecylamin), Margarylamin (1-Heptadecylamin), Stearylamin (1-Octadecylamin), Arachidylamin (1-Eicosylamin), Behenylamin (1-Docosylamin), Lignocerylamin (1-Tetracosylamin), Cerylamin (1-Hexacosylamin), Montanylamin (1-Octacosylamin), Melissylamin (1-Triacontylamin), Palmitoleylamin (cis-9-Hexadecenylamin), Oleylamin (cis-9-Octadecenylamin) und/oder Elaidylamin (trans-9-Octadecenylamin) sowie Mischungen dieser Substanzen, wobei Cetylamin und/oder Stearylamin sowie Mischungen dieser beiden Substanzen besonders bevorzugt sind.

6. Verwendung nach Anspruch 1 oder 2, **dadurch gekennzeichnet, dass** das zweischwänzige, langkettige Aniontensid der allgemeinen Formel (II) entspricht,
R²-Aⁿ⁻ n/m Kat.^{m+} Formel (II)
wobei R² ein verzweigter Kohlenwasserstoffrest ist, welcher wiederum aus zwei gleichen oder verschiedenen einwertigen aliphatischen oder aromatischen, gesättigten oder ungesättigten Kohlenwasserstoffresten mit je 12 bis 40 C-Atomen, bevorzugt 14 bis 30, besonders bevorzugt mit 16 bis 24 Kohlenstoffatomen besteht, und wobei Aⁿ⁻ die anionische Kopfgruppe des Tensids ist, welche n negative Ladungen trägt, wobei n = 1 - 3, mehr bevorzugt 1 - 2, noch mehr bevorzugt gleich 1, und wobei Kat.^{m+} das kationische Gegenion ist, welches n positive Ladungen trägt, wobei m = 1 - 10, bevorzugt 1 - 5, mehr bevorzugt 1 - 2, noch mehr bevorzugt gleich 1.

7. Verwendung nach mindestens einem der Ansprüche 1 - 6 **dadurch gekennzeichnet, dass** das zweischwänzige, langkettige Aniontensid der allgemeinen Formel (III) oder (IV) entspricht, 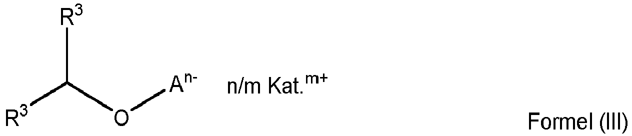 bzw. 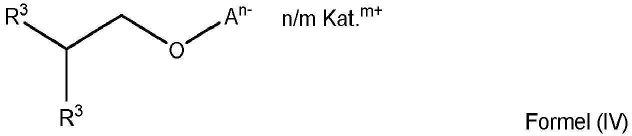 wobei die Reste R³ unabhängig voneinander gleiche oder verschiedene einwertige aliphatische oder aromatische, gesättigte oder ungesättigte Kohlenwasserstoffrest mit 12 bis 40 C-Atomen, bevorzugt 14 bis 30, besonders bevorzugt mit 16 bis 24 Kohlenstoffatomen sind, und wobei Aⁿ⁻ die anionische Kopfgruppe des Tensids ist, welche n negative Ladungen trägt, wobei n = 1 - 3, mehr bevorzugt 1 - 2, noch mehr bevorzugt gleich 1, und wobei Kat.^{m+} das kationische Gegenion ist, welches m positive Ladungen trägt, wobei m = 1 - 10, bevorzugt 1 - 5, mehr bevorzugt 1 - 2, noch mehr bevorzugt gleich 1.

8. Verwendung nach mindestens einem der Ansprüche 1 bis 7 **dadurch gekennzeichnet, dass** die anionischen Kopfgruppe Aⁿ⁻ auf organischen Carboxylaten oder anionischen Schwefel- oder Phosphorverbindungen basiert, wobei die Kopfgruppe in diesem Zusammenhang bevorzugt Struktureinheiten ausgewählt aus der Gruppe der Carboxylate, der Phosphate, der Phosphonate, der Phosphinate, der Sulfate sowie der Sulfonate aufweist.

9. Verwendung nach mindestens einem der Ansprüche 1 bis 8 **dadurch gekennzeichnet, dass** das zweischwänzige, langkettige Aniontensid ausgewählt ist aus der Gruppe der Dialkyl-Sulfosuccinate, wobei Dipalmityl-Sulfosuccinat, Distearyl-Sulfosuccinat und/oder Stearyl-Palmityl-Sulfosuccinat besonders bevorzugt sind, der Gruppe der Dialkyl-Sulfosuccinamate, wobei Dipalmityl-Sulfosuccinamat, Distearyl-Sulfosuccinamat und/oder Stearyl-Palmityl-Sulfosuccinamat besonders bevorzugt sind, der Gruppe der Dialkyl-Phosphate, wobei Dipalmityl-Phosphat, Distearyl-Phosphat und/oder Stearyl-Palmityl-Phosphat besonders bevorzugt sind, der Gruppe der Dialkyl-Ester Tri- oder höherfunktionaler Carbonsäuren, wobei Distearyl-Ester von Tri- oder höherfunktionaler Carbonsäuren, Stearyl-Palmityl-Ester von Tri- oder höherfunktionaler Carbonsäuren besonders bevorzugt sind, der Gruppe der Monoestervon Phosphorsäure, Schwefelsäure sowie Di- oder höherwertigen Carbonsäuren mit langkettigen verzweigten Alkoholen, der Gruppe der Monoalkyl- Sulfosuccinate, welche auf langkettigen verzweigten Alkoholen basieren, sowie Mischungen dieser Substanzen.

10. Verwendung nach mindestens einem der Ansprüche 1 bis 9 **dadurch gekennzeichnet, dass** das Kation Kat.^{m+} ein Metall-Kationen, besonders bevorzugt ein Lithium-, Natrium-, Kalium-, Calcium-, Strontium-, Barium-, Barium- oder Silber-Kationen ist, wobei Natrium oder Kalium-Kationen ganz besonders bevorzugt sind, und/oder ein Ammonium-Kation einer protonierten Amin-Verbindung ist, wobei Ammonium-Ionen auf Basis von Ammoniak, Aminen, wie Mono-, Di- und Trialkylamine, wobei es sich auch um funktionalisierte Alkylreste wie beispielsweise bei Amidaminen, Mono-, Di- und Trialkanolamine oder Mono-, Di- und Triaminoalkylamine handeln kann, sowie höherwertigen Aminen, wie z.B. Ethylendiamin, Diethylentriamin oder Triethylentetraamin besonders bevorzugt sind und/oder ein Hydronium-Ion, bzw. im Falle der nicht dissoziierten Säure ein an die Kopfgruppe gebundenes Wasserstoffatom ist.

11. Verwendung nach mindestens einem der Ansprüche 1 bis 10 **dadurch gekennzeichnet, dass** die zweischwänzigen, langkettigen Aniontenside in Kombination mit mindesten einem weiteren Co-Tensid als Additiv in wässrigen Polymerdispersionen, bevorzugt wässrigen Polyurethandispersionen eingesetzt werden, wobei es sich bei dem Co-Tensid bevorzugt um Fettalkohole, Fettsäureamide, Ethylenoxid-Propylenoxid-Blockcopolymere, Betaine, wie z.B. Amido-Propyl-Betaine, Aminoxide, quaternäre Ammonium-Tensid, Amphoacetate Ammonium und/oder Alkali-Salze von Fettsäure, Alkylsulfate, Alkylethersulfate, Alkylsulfonate, Alkylbenzolsulfonate, Alkylphosphate, Alkyl-Sulfosuccinate, Alkyl-Sulfosuccinamate, Alkylsarcosinate oder auch silikon-basierte Co-Tenside sowie Mischungen dieser Substanzen handelt, wobei freie Fettalkohole, bevorzugt mit 12 bis 40, mehr bevorzugt mit 14 - 30, noch mehr bevorzugt mit 16 - 24 Kohlenstoffatomen, sowie Alkylsulfate mit 12 bis 40, mehr bevorzugt mit 14 - 30, noch mehr bevorzugt mit 16 - 24, sowie Mischungen dieser Substanzen ganz besonders bevorzugt sind.

12. Verwendung nach einem der Ansprüche 1 bis 11, **dadurch gekennzeichnet, dass** die wässrigen Polymer-Dispersionen ausgewählt sind aus der Gruppe wässriger Polystyrol-Dispersionen, Polybuatien-Dispersionen, Poly(meth)acrylat-Disperionen, Polyvinylester-Dispersionen und Polyurethan-Dispersionen, insbesondere Polyurethan-Dispersionen, und wobei der Festkörper-Anteil dieser Dispersionen bevorzugt im Bereich von 20 - 70 Gew.-% liegt, mehr bevorzugt im Bereich von 25 - 65 Gew.-%, bezogen auf die gesamte Dispersion.

13. Verwendung nach mindestens einem der Ansprüche 1 bis 12, **dadurch gekennzeichnet, dass** die Konzentration der zweischwänzigen, langkettigen Aniontensiden bezogen auf das Gesamtgewicht der wässrigen Polymerdispersion im Bereich von 0,1 - 20 Gew.-%, besonders bevorzugt im Bereich von 0,2 - 15 Gew.-%, insbesondere bevorzugt im Bereich von 0,5 - 10 Gew.-% liegt.

14. Wässrige Polymerdispersion, vorzugsweise wässrige Polyurethan-Dispersion, enthaltend zweischwänzige, langkettige Aniontenside, vorzugsweise wie in den Ansprüchen 1 bis 13 beschrieben, wobei der Festkörper-Anteil dieser Dispersionen bevorzugt im Bereich von 20 - 70 Gew.-% liegt, mehr bevorzugt im Bereich von 25 - 65 Gew.-%, bezogen auf die gesamte Dispersion, und wobei die Konzentration der zweischwänzigen, langkettigen Aniontensiden bezogen auf das Gesamtgewicht der wässrigen Polymerdispersion im Bereich von 0,2 - 20 Gew.-%, besonders bevorzugt im Bereich von 0,4 - 15 Gew.-%, insbesondere bevorzugt im Bereich von 0,5 - 10 Gew.-% liegt.

15. Verfahren zur Herstellung einer porösen Polymerbeschichtung, vorzugsweise porösen Polyurethanbeschichtung unter Verwendung von zweischwänzigen, langkettigen Aniontenside als Additive in wässrigen Polymer-Dispersionen, vorzugsweise wässrigen Polyurethan-Dispersionen, vorzugsweise wie in den Ansprüchen 1 bis 13 beschrieben, umfassend die Schritte
a) Bereitstellen einer Mischung enthaltend mindestens eine wässrige Polymerdispersion, mindestens ein erfindungsgemäßes zweischwänziges, langkettiges Aniontensid, sowie ggf. weitere Additive
b) Aufschäumen der Mischung zu einem Schaum
c) optional Zugabe mindestens eines Verdickers zur Einstellung der Viskosität des Nassschaums
d) Auftragen einer Beschichtung der geschäumten Polymer-Dispersion, vorzugsweise Polyurethan-Dispersion, auf einen geeigneten Träger,
e) Trocknen der Beschichtung.

16. Poröse Polymerbeschichtung, vorzugsweise poröse Polyurethanbeschichtung, erhältlich durch den Einsatz von zweischwänzigen, langkettigen Aniontensiden als Additive in wässrigen Polymer-Dispersionen, vorzugsweise wässrigen Polyurethan-Dispersionen, bei der Herstellung solcher Polymerbeschichtungen, bevorzugt erhältlich durch ein Verfahren nach Anspruch 15.

17. Gebrauchsgegenstände enthaltend eine poröse Polymerbeschichtung nach Anspruch 16, wobei es sich dabei bevorzugt um Schuhe, Einlegesohlen, Taschen, Koffer, Etuis, Kleidung, Automobilteile, bevorzugt Sitzbezüge, Bespannungen von Türteilen, Armaturenbrettteilen, Lenkrädern und/oder Griffen sowie Schaltsäcken, Einrichtungsgegenstände wie Schreibunterlagen, Kissen oder Sitzmöbeln, Gap Filler in elektronischen Geräten, Polster- und Dämpfungsmaterialien in medizinischen Anwendungen oder Klebebänder handelt.
